# EUROPÄISCHE PATENTANMELDUNG

(11) **EP 2 786 772 A1**
(43) Veröffentlichungstag der Anmeldung: **08.10.2014**
(21) Anmeldenummer: 14168573.5
(22) Anmeldetag: 26.02.2008
(51) Int. Cl.: A61M 1/10, A61M 1/16, A61M 1/26

(54) **Vorrichtung für den Stoff- und/oder Energieaustausch**

(30) Priorität: 28.02.2007 DE 102007010112
(62) Teilanmeldung aus: 08716030.5
(71) Anmelder: Dritte Patentportfolio Beteiligungsgesellschaft mbH & Co. KG, 12529 Schönefeld/OT Waltersdorf (DE)
(72) Erfinder: Kashefi Khorasani, Ahmad Ali, 52074 Aachen (DE); Oedekoven, Bernward, 6351 AP Bocholtz (NL); Dautzenberg, Rainer, 52072 Aachen (DE)
(74) Vertreter: Zech, Stefan Markus

(57) **Zusammenfassung**

Die Erfindung betrifft eine Vorrichtung für den Stoff- und/oder Energieaustausch zwischen zwei Medien, insbesondere zwischen Blut und einem Gas/Gasgemisch, mit einer von dem ersten Medium durchströmten Kammer (2), in der wenigstens eine stoff- und/oder energie-permeable Hohlfaser (5), bevorzugt eine Vielzahl von stoff- und/oder energie-permeablen Hohlfasern (5) angeordnet ist, die von dem zweiten Medium durchströmbar und von dem erstem Medium umströmbar ist, bei der in der Kammer (2) wenigstens ein Pumpelement (5) angeordnet ist, mittels dem das erste Medium aus der Kammer (2) verdrängbar und/oder in die Kammer (2) hinein saugbar ist.

## Beschreibung

Die Erfindung betrifft eine Vorrichtung für den Stoff- und/oder Energieaustausch zwischen zwei Medien, insbesondere zwischen Blut und einem Gas/Gasgemisch, mit einer von dem ersten Medium durchströmten Kammer, in der wenigstens eine stoff- und/oder energie-permeable Hohlfaser, bevorzugt eine Vielzahl von stoff- und/oder energie-permeablen Hohlfasern angeordnet ist, die von dem zweiten Medium durchströmbar und von dem ersten Medium umströmbar ist.

Derartige gattungsgemäße Vorrichtungen finden ihr Einsatzgebiet beispielsweise in der Medizintechnik und hier insbesondere bei den Anwendungen der Blutreinigung, wie z.B. der Dialyse, der Bluttrennungen oder auch der künstlichen Lungen (Oxygenatoren).

Im Anwendungsgebiet der Oxygenatoren ist es dabei vorgesehen, das Blut als ein erstes Medium durch eine Kammer strömen zu lassen, in der wenigstens eine stoff- und/oder energie-permeabie Hohlfaser, in bevorzugter Ausführung eine Vielzahl von stoff- und/oder energie-permeablen Hohlfasern angeordnet ist, die von dem zweiten Medium, insbesondere hier Sauerstoff, durchströmbar ist und von dem ersten Medium umströmt wird.

Wird bei dieser Ausführung aus dem Körper eines Lebewesens stammendes mit CO₂ angereichertes Blut durch die Kammer gepumpt, so ergibt sich durch die unterschiedlichen Partialdrücke von Sauerstoff und CO₂ auf den beiden Seiten der stoff- und/oder energie-permeablen Hohlfaser ein Stoffaustausch in dem Sinne, dass CO₂ aus dem Blut entfernt und dieses mit Sauerstoff aus den Hohlfasern angereichert wird. So kann eine derartige Vorrichtung als künstliche Lunge arbeiten und beispielsweise die Lungenfunktion eines Patienten teilweise oder auch vollständig übernehmen.

Soweit in dieser Erfindungsbeschreibung ein Oxygenator, d.h. eine künstliche Lunge, näher beschrieben wird, ist dies nicht als Einschränkung, sondern lediglich als beispielhafte Anwendung zu verstehen. Die im späteren erfindungsgemäß beschriebene Vorrichtung kann grundsätzlich für den Stoff- bzw. Energieaustausch zwischen beliebigen Medien eingesetzt werden und dies nicht nur in der Medizintechnik, sondern auch bei anderen industriellen Anwendungen.

Zur Erzielung adäquater und definierter Flussraten eines ersten Mediums durch die genannte Kammer, insbesondere des Blutes durch die Kammer, werden externe Pumpen benötigt. Im Anwendungsbereich der Oxygenatoren bedeutet dies, dass zusätzlich zu der gattungsgemäßen Vorrichtung eine externe Pumpe vorgesehen sein muss, mit der sichergestellt wird, dass Blut aus dem Körper eines Patienten durch die Vorrichtung und sodann zurück in den Körper des Patienten gepumpt wird. Hierbei wird unter dem Körper eines Patienten sowohl der Körper eines menschlichen als auch eines tierischen Patienten verstanden.

Dieses genannte Prinzip bedingt, dass bei der gesamten Betrachtung der gattungsgemäßen Vorrichtungen sowie der extern eingesetzten Pumpen sich ein erhebliches Volumen ergibt, welches durch das erste Medium, wie in dieser Anwendung beispielsweise das Blut, gefüllt werden muss.

Insbesondere bei Babys und hier speziell bei Frühgeborenen, die ein sehr geringes Blutvolumen, teilweise unter 100 Milliliter haben, bedeutet dies, dass die gattungsgemäß bekannten Vorrichtungen nicht zum Einsatz kommen können bzw. der Organismus eines Babys stark belastet wird durch das Hinzufügen von Fremdblut, Blutplasma oder Plasmaexpander, um das Volumen der bekannten Vorrichtungen füllen zu können. Darüber hinaus ist der Einsatz derartiger gattungsgemäß bekannter Vorrichtungen aufgrund der zusätzlichen Pumpen auch nur außerhalb des Körpers eines Patienten möglich.

Aufgabe der Erfindung ist es, eine gattungsgemäß bekannte Vorrichtung derart weiterzubilden, dass eine kleinbauende Ausführungen erreicht wird, insbesondere die nur ein minimales Füllvolumen aufweist und darüber hinaus auch der Einsatz als implantierbare Vorrichtung erschlossen wird.

Gelöst wird diese Aufgabe durch eine Vorrichtung der eingangs genannten gattungsgemäßen Art, bei der in der genannten Kammer eine Pumpfunktion vorgesehen ist.

Beispielsweise kann hierfür ein Pumpelement in die Vorrichtung, insbesondere in die Kammer integriert sein beispielsweise durch wenigstens ein verformbares, insbesondere elastisch verformbares Element, dessen Oberfläche/Volumen zumindest bereichsweise änderbar ist, so dass bei einer Oberflächen-/Volumenvergrößerung das erste Medium aus der Kammer verdrängbar und bei einer Oberflächen-/Volumenverkleinerung das erste Medium in die Kammer hinein saugbar ist.

Der wesentliche Kerngedanke dieses erfindungsgemäßen Aspektes ist es, dass eine Pumpfunktion durch diese Ausführung unmittelbar in die Vorrichtung integriert werden kann und somit eine extrakorporale zusätzliche Pumpe entfallen kann. Hierbei wird der Pumpeffekt dadurch erzielt, dass durch die Vergrößerung der Oberfläche eines verformbaren, insbesondere elastisch verformbaren Elementes, und der damit einhergehenden Volumenvergrößerung automatisch das in der genannten Kammer befindliche erste Medium, wie beispielsweise Blut, verdrängt wird und bei einer dann folgenden Oberflächen- bzw. Volumenverkleinerung dieses Elementes aufgrund des entstehenden Unterdruckes das erste Medium, also beispielsweise Blut, in die Kammer wiederum hineingesaugt wird.

Hierbei ist sicherzustellen, dass durch den Vorgang des Verdrängens immer das durch den Stoff- bzw. Energieaustausch wunschgemäß behandelte erste Medium verdrängt und beim Ansaugen das noch nicht behandelte Medium angesaugt wird. Dies kann dadurch erzielt werden, dass eine Flussrichtung durch die Kammer definiert wird, was in einer bevorzugten Ausführungsform der Erfindung dadurch erfolgen kann, dass im Flussweg zumindest ein Einwege-Ventil, bevorzugt in der Kammer am Eingang und am Ausgang für das erste Medium jeweils ein Einweg-Ventil angeordnet ist.

Ein derartiges Ventil kann nur in einer Richtung durch das erste Medium durchflossen werden, so dass bei Ausführung mit zwei Ventilen die Ansaugung des ersten Mediums immer durch das eine Ventil und das Verdrängen des ersten Mediums immer durch das andere Ventil erfolgen kann. Die Anordnung dieser Ventile unmittelbar in der Kammer bzw. an dem Ein- bzw. Ausgang der Kammer führt damit weiterhin zu einer Verringerung der Baugröße sowie zu einer bevorzugten Kompaktheit der erfindungsgemäßen Vorrichtung, da derartige Hilfsmittel zur Definition der Flussrichtung in diesem Fall nicht mehr extern vorgesehen sein müssen.

Das verformbare Element kann dabei beispielsweise von außerhalb der Vorrichtung angesteuert werden, wofür verschiedene Maßnahmen zur Verfügung stehen können. Beispielsweise kann es hier vorgesehen sein, dass das verformbare Element seine Form und damit seine Oberflächengröße bzw. das Volumen schon alleine dadurch ändert, dass es elektrisch bzw. elektronisch und/oder hydraulisch und/oder pneumatisch angesteuert wird.

Z.B. kann das verformbare Element als ein Ballon ausgebildet sein und somit eine inflatierbare und deflatierbare Hülle bilden, die ergänzend auch elastisch ausgebildet sein kann. Z.B. durch periodisches Füllen und Entleeren mittels eines Fluids (Gas oder Flüssigkeit) kann so der Pumpeffekt erzielt werden.

In einer besonders bevorzugten Ausführung kann es hier auch vorgesehen sein, dass das wenigstens eine verformbare Element als verformbare, insbesondere elastische verformbare Hohlfaser ausgebildet ist, deren Oberfläche/Volumen zumindest bereichsweise durch innere Beaufschlagung mit einem Medium änderbar ist. Ist beispielsweise eine derartige insbesondere elastische Hohlfaser durch ein fluides Medium, also z.B. ein Gas oder auch eine Flüssigkeit gefüllt, so kann durch Änderung des Druckes dieses Mediums bewirkt werden, dass sich die Oberfläche und damit das durch die Hohlfaser in der Kammer beanspruchte Volumen vergrößert. Auch hierdurch kann demnach der gewünschte Pumpeffekt gemäß der Erfindung erzielt werden. Desweiteren kann durch Erzeugung eines Unterdruckes innerhalb der Hohlfaser das Volumen in der Kammer vergrößert werden. So kann bei Beaufschlagung der Hohlfaser mit Druck ein vergrößertes "Schlagvolumen" erreicht werden.

Hierbei kann es gemäß einer Ausführung der Erfindung vorgesehen sein, dass in der Vorrichtung stoff- und/oder energie-permeable Hohlfasem, insbesondere zumindest wenigstens eine vorgesehen sind, die nur für den Stoff- bzw. Energieaustausch eingesetzt werden und das wenigstens eine elastisch verformbare Hohlfaser zum Einsatz kommt, die für die Pumpzwecke in die Vorrichtung integriert ist. Hierbei können sowohl von den Hohlfasern, die zum Stoff- oder Energieaustausch eingesetzt werden, eine oder mehrere zum Einsatz kommen, ebenso wie auch bei der Hohlfaser, die für die Pumpzwecke verwendet wird. Bei den stoff- und/oder energie-permeable Hohlfasern kann es sich auch um starre Hohlfasern handeln.

In einer anderen bevorzugten Ausführungsform kann es auch vorgesehen sein, dass die wenigstens eine elastisch verformbare Hohlfaser als stoff- und/oder energie-permeable Hohlfaser ausgebildet ist. Beispielsweise kann hierfür eine elastisch verformbare und stoff- und/oder energie-permeable Hohlfaser als Silikonschlauch ausgebildet sein, da Silikon, insbesondere in der Anwendung als Oxygenator, für Sauerstoff und Kohlendioxid permeabel ist, so dass durch einen Silikonschlauch sowohl der Gasaustausch zwischen der Gasphase und der Blutphase einer erfindungsgemäßen Vorrichtung stattfinden kann, ebenso wie durch die elastische Verformbarkeit auch die Pumpfunktion realisiert werden kann. So kann es in einer besonders einfachen und demnach auch kleinbauenden und günstigen Ausführungsform vorgesehen sein, dass eine Vorrichtung nur eine einzige, insbesondere elastisch verformbare und gleichzeitig stoff- und/oder energie-permeable Hohlfaser aufweist, die sowohl die Pumpfunktion des ersten Mediums übernimmt als auch das zweite Medium transportiert, mittels dem ein Stoff- und/oder Energieaustausch stattfinden soll. Selbstverständlich können hier statt einer insbesondere elastischen verformbaren und stoff- und/oder energie-permeablen Hohlfaser auch beliebig viele energie-permeable verformbare Hohlfasern eingesetzt werden. Die Vorrichtung kann dann nur Hohlfasern der elastischen und permeablen Art umfassen. Bei dieser Ausführung kann es demnach vorgesehen sein, dass das Medium zur Beaufschlagung der wenigstens einen elastisch verformbaren Hohlfaser dem zweiten Medium entspricht.

In einer anderen Ausführung kann es auch vorgesehen sein, dass wenigstens ein Teil von allen stoff- und/oder energie-permeablen Hohlfasern auch als elastisch verformbare Hohlfasern ausgebildet ist. So kann es bei einer solchen Ausführung beispielsweise solche stoff- und/oder energie-permeablen Hohlfasern geben, die als starre Hohlfasern ausgebildet sind, genauso wie stoff- und/oder energie-permeable Hohlfasern, die wie eingangs erwähnt elastisch verformbar sind. Hierbei kann von jeder Art der Hohlfasern eine oder mehrere vorgesehen sein.

In wiederum anderer Ausführung können die elastisch verformbaren Hohlfasern lediglich zu Pumpzwecken eingesetzt werden, insbesondere ohne dass diese selbst stoff-permeabel, gegebenenfalls jedoch energie-permeabel sind.

So kann es demnach insbesondere bei den zuletzt genannten Ausführungen mit zwei verschiedenen Arten von Hohlfasern (Pump-Fasern und Stoff-Austauschfasern) vorgesehen sein, dass das Medium zur Beaufschlagung der wenigstens einen elastisch verformbaren Hohlfaser, also das Medium, welches zur Realisierung der Pumpfunktion verwendet wird, nicht dem zweiten Medium entspricht, sondern durch ein weiteres fluides Medium, wie beispielsweise ein Gas oder eine Flüssigkeit gebildet wird. Hierbei kann es ergänzend vorgesehen sein, dass durch die Verwendung dieses Mediums auch ein Wärmeaustausch zum ersten Medium realisiert wird.

Dies ist beispielsweise in der Anwendung bei Blutoxygenatoren besonders vorteilhaft, da hierbei neben dem Pumpeneffekt, der durch das weitere bzw. zusätzliche Medium realisiert wird, auch sichergestellt werden kann, dass genügend Wärmeenergie an das erste Medium, in dieser Anwendung also hier das Blut übertragen wird, um die Körpertemperatur des Patienten aufrecht zu erhalten. Da das erste Medium, in dieser Anwendung das Blut, zumindest in dem Bereich der elastischen Hohlfaser, in welchem eine Oberflächen- bzw. Volumenvergrößerung stattfindet, diese Hohlfaser umströmt, kann demnach gleichzeitig ein Wärmeenergietransfer zum ersten Medium erfolgen.

Die Erzeugung einer Pumpfunktion innerhalb der Kammer der Vorrichtung, welche vom ersten Medium, also beispielsweise vom Blut durchströmt wird, hat neben dem reinen Pumpeffekt noch den weiteren Vorteil, dass eine Störung durch die Druckschwankungen in der Kammer erzeugt wird, wodurch ein Plasmasaum, der sich kammerseitig an den stoff-permeablen Hohlfasern ausbilden kann, zerstört oder zumindest gestört werden kann, um hier die Effizienz der Stoff- und/oder Energieübertragung zu optimieren.

In einer weiteren erfindungsgemäß bevorzugten Ausführung kann es auch vorgesehen sein, dass zusätzlich zu der genannten Kammer, die vom ersten Medium durchströmt ist, wenigstens eine weitere Kammer vorgesehen ist, über welche das zweite Medium der wenigstens einen stoff- und/oder energie-permeablen Hohlfaser, bevorzugt der Vielzahl von stoff- und/oder energie-permeablen Hohlfasern zugeführt ist, wobei sich die wenigstens eine elastisch verformbare Hohlfaser durch die genannte und die wenigstens eine weitere Kammer erstreckt.

Hierdurch ergibt sich der Vorteil, dass durch die Erstreckung der verformbaren Hohlfaser auch durch die weitere Kammer gegebenenfalls auch ein Wärmeaustausch, insbesondere eine Wärmeübertragung zum zweiten Medium stattfinden kann, so dass eine Wärmeübertragung nach Erwärmung des zweiten Mediums auch durch die stoff-permeablen Hohlfasern zum ersten Medium stattfinden kann.

Weiterhin kann die Ausführung derart sein, dass die Oberfläche/das Volumen der wenigstens einen insbesondere elastisch verformbaren Hohlfaser auch in der wenigstens einen weiteren Kammer durch innere Beaufschlagung mit dem Medium zumindest bereichsweise änderbar ist, so dass bei einer Oberflächen-Nolumenänderung Druckschwankungen in dem zweiten Medium erzeugbar sind. Diese Druckschwankungen können vorteilhafterweise bewirken, dass eine Gasgrenzschicht, die sich seitens der Phase des zweiten Mediums bzw. seitens der Gasphase an der stoff-permeablen Hohlfaser ausbildet, gestört bzw. zerstört wird, so dass auch hierdurch der Stoff- und/oder Energieaustausch verbessert bzw. optimiert wird.

Bei sämtlichen der eingangs genannten Ausführungen, seien diese mit nur einer oder auch mehreren Kammern, kann es vorgesehen sein, dass die Oberflächen-/Volumenänderung der wenigstens einen insbesondere elastisch verformbaren Hohlfaser durch Druckänderungen des durch diese Hohlfaser fließenden Mediums erzeugbar sind.

Hierbei kann es sich wie eingangs genannt um ein fluides Medium, wie beispielsweise Gas oder auch eine Flüssigkeit handeln. Die Druckänderungen können hierbei erzeugbar sein durch einen kontinuierlichen Zufluss dieses Mediums in die wenigstens eine insbesondere elastisch verformbare Hohlfaser am Eingang in die Vorrichtung und ein in der Menge steuerbaren Abfluss am Austritt der Vorrichtung, insbesondere durch einen gesteuert änderbaren Querschnitt.

So kann der gesteuert änderbare Querschnitt am Abfluss gezielt reduziert oder sogar geschlossen werden, wodurch bei einem kontinuierlichen Zufluss eingangsseitig sich der Druck in der insbesondere elastisch verformbaren Hohlfaser vergrößert, wodurch diese auf Grund der elastischen Verformung ihr in der Kammer eingenommenes Volumen vergrößert und das erste Medium, hier insbesondere Blut, verdrängt. Wird der gesteuert änderbare Querschnitt vergrößert, insbesondere nach einer vorherigen Schließung geöffnet, so wird der Druck durch z.B. elastische rückstellende Kraft abgebaut und das Volumen der Hohlfaser kann sich verringern, so dass das ursprünglich verdrängte Volumen des ersten Mediums kompensiert wird durch das Nachsaugen vom ersten Medium in die Kammer.

Bei sämtlichen Ausführungen kann es vorgesehen sein, dass eine insbesondere elastisch verformbare Hohlfaser über ihre gesamte Länge, welche diese in der zumindest einen Kammer der Vorrichtung einnimmt, verformbar ist. In einer anderen Ausführung kann es auch vorgesehen sein, dass bewusst die Änderung der Oberflächengröße bzw. des Volumens nur auf bestimmte Bereiche, insbesondere wenigstens einen Bereich der elastisch verformbaren Hohlfaser begrenzt wird. Beispielweise kann zur Erzeugung wenigstens eines lokal begrenzten Bereiches dieser Oberflächen- bzw. Volumenänderung eine verformbare, insbesondere elastische Hohlfaser wenigstens ein sie zumindest teilweise umgebendes Stützelement aufweisen. Bei einem solchen Stützelement kann es sich beispielsweise um einen Ring, ein Rohr oder auch ein Gitter handeln, welches die elastische Hohlfaser zumindest teilweise umgibt, so dass in dem umgebenen Bereich keine Volumen- bzw. Oberflächenvergrößerung erfolgen kann, sondern lediglich in den Bereichen, die von dem stützenden Element frei bleiben.

In einer anderen Ausführung kann es auch vorgesehen sein, dass zur Erzeugung wenigstens eines lokal begrenzten Bereiches der Oberflächen- bzw. Volumenänderung eine elastische Hohlfaser, eine bereichsweise variable Wandstärke und/oder eine Oberflächenprofilierung aufweist. Ist beispielsweise die Wandstärke in einem bestimmten Bereich gegenüber anderen Bereichen verringert, so wird die elastische Hohlfaser dazu tendieren, sich maßgeblich in diesem dickenreduzierten Bereich auszubeulen auf Grund eines vom Inneren der Faser wirkenden Druckanstieges.

Durch alle diese vorgenannten Maßnahmen kann demnach das Verdrängungsvolumen einer elastischen Hohlfaser genauestens definiert werden.

Hierbei kann es ergänzend auch weiterhin vorgesehen sein, dass eine insbesondere elastisch verformbare Hohlfaser, beispielsweise in einem Käfig angeordnet ist, welcher das maximal verdrängte Volumen begrenzt. So kann sich bei einem Aufblähen einer Hohlfaser diese nur soweit ausdehnen und im Volumen vergrößern, bis dass die Wandbereiche der elastischen Hohlfaser an den Innenbereichen des umgebenden Käfigs anliegen. Die Hohlfaser wird sodann durch den Käfig gestützt und kann sich nicht weiter ausdehnen, so dass sich das effektiv verdrängte Volumen durch das Maximalvolumen des umgebenden Käfigs umgibt.

Auch kann eine Hohlfaser einen nicht elastischen verformbaren Abschnitt aufweisen, der durch innere Druckbeaufschlagung nicht elastisch aufblähbar ist, wie z.B. in der Art einer Plastiktüte, bis dass das Maximalvolumen dieses Bereiches erreicht ist. Durch rückstellende Kräfte, insbesondere Federkräfte kann dieser Bereich bei Druckabnahme wieder aktiv zusammenfallen, insbesondere zusammengefaltet werden. Hierzu können z.B. Federelemente (wenigstens eines) in der Wandung des verformbaren Abschnittes angeordnet sein.

Die Anordnung der wenigstens einen insbesondere elastisch verformbaren Hohlfaser und der Stoff- und/oder energie-permeablen Hohlfasern, die gegebenenfalls gemäß einer Ausführung auch miteinander identisch sein können, kann grundsätzlich beliebiger Art sein.

Es wird jedoch erfindungsgemäß als besonders vorteilhaft empfunden, wenn wenigstens eine elastisch verformbare Hohlfaser von einer Vielzahl von nicht-elastischen und stoff- und/oder energie-permeablen Hohlfasern, insbesondere symmetrisch umgeben ist. Auch hier kann die umgebene verformbare Hohlfaser selbst ebenso stoff- oder energie-permeabel ausgebildet sein oder gegebenenfalls nur für die Pumpfunktion vorgesehen sein. Diese genannte Anordnung kann bevorzugterweise derart ausgeführt werden, dass die Gesamtanordnung von Hohlfasern einen im Wesentlichen mehreckigen, insbesondere sechseckigen Querschnitt aufweist.

Zur Erzielung einer besonders hohen Packungsdichte kann es dabei vorgesehen sein, dass die nicht-elastischen und stoff- und/oder energie-permeablen Hohlfasern versetzt zueinander angeordnet sind, insbesondere um einen halben Abstand zwischen zwei Hohlfasern versetzt geschichtet sind. Bei einer solchen Ausführung können beispielsweise die stoff- und/oder energie-permeablen Hohlfasern als Matten ausgebildet sein, die aufeinander geschichtet werden, hier insbesondere um den halben Abstand versetzt, so dass eine Hohlfaser einer zweiten Matte, die auf eine erste Matte aufgelegt wird genau zwischen zwei Hohlfasern der ersten Matte zu liegen kommt. Hierdurch ergibt sich die eingangs erwähnte hohe Packungsdichte.

Die erfindungsgemäße Vorrichtung hat den besonderen Vorteil, dass eine hohe Effizienz, insbesondere auch durch die erfindungsgemäßen und eingangs beschriebenen Störungen der Plasmasäume bzw. Gasgrenzschichten beiderseits der stoff-permeablen Hohlfasern erreicht wird, wobei auf Grund der hohen Effizienz und der integrierten Pumpfunktion eine sehr kleinbauende Form erreicht werden kann, insbesondere mit der Füllvolumina des ersten Mediums in der Vorrichtung von weniger als 100 ml realisierbar sind.

Derartige Vorrichtungen können daher in besonders bevorzugter Weise beispielsweise als Blut-Oxygenator, insbesondere für Babys und/oder Frühgeborene zum Einsatz kommen und auch als implantierbare Oxygenatoren verwendet werden, beispielsweise wenn die Funktion eines Lungenflügels unterstützt oder sogar ganz ersetzt werden muss.

Darüber hinaus bietet es sich auch an, die erfindungsgemäße Vorrichtung als beliebiges implantierbares künstliches Organ, beispielsweise auch als Dialysegerät (künstliche Niere) oder auch als künstliche Leber einzusetzen. Hierbei ist lediglich an Hand der eingesetzten ersten und zweiten gegebenenfalls auch der dritten Medien sowie der eingesetzten stoff- und/oder energie-permeablen Hohlfasern zu definieren, für welche Art von Stoffaustausch die Vorrichtung geeignet sein soll.

Ein Ausführungsbeispiel des Standes der Technik sowie Ausführungsbeispiele der Erfindungen werden nachfolgend näher beschrieben. Es zeigen:
- Figur 1: den prinzipiellen Aufbau eines im Stand der Technik bekannten Blut-Oxygenators,
- Figur 2: den grundsätzlichen Aufbau eines erfindungsgemäßen Blut-Oxygenators mit nur einer stoff-permeablen Hohlfaser, die gleichzeitig auf Grund ihrer elastischen Verformbarkeit als Pumpe dient,
- Figur 3: die Ausführungsform eines Zweikammermodells eines Blut-Oxygenators,
- Figur 4: die prinzipielle Darstellung des Sauerstoffaustausches in einem Blut-Oxygenator an der Grenzschicht einer mikroporösen Membran,
- Figur 5: die Ausführung einer elastisch verformbaren Hohlfaser mit partiell reduzierter Wandstärke.
- Figur 6: eine mögliche Gehäuseform

Im Folgenden wird der Stand der Technik und erfindungsgemäße Ausführungen am Beispiel eines Blut-Oxygenators beschrieben, um die erfindungsgemäßen und wesentlichen Vorteile herauszustellen. Dieses Beispiel ist nicht beschränkend und in analoger Weise auf den Stoff- bzw. Energieaustausch auch zwischen anderen Medien zu verstehen.

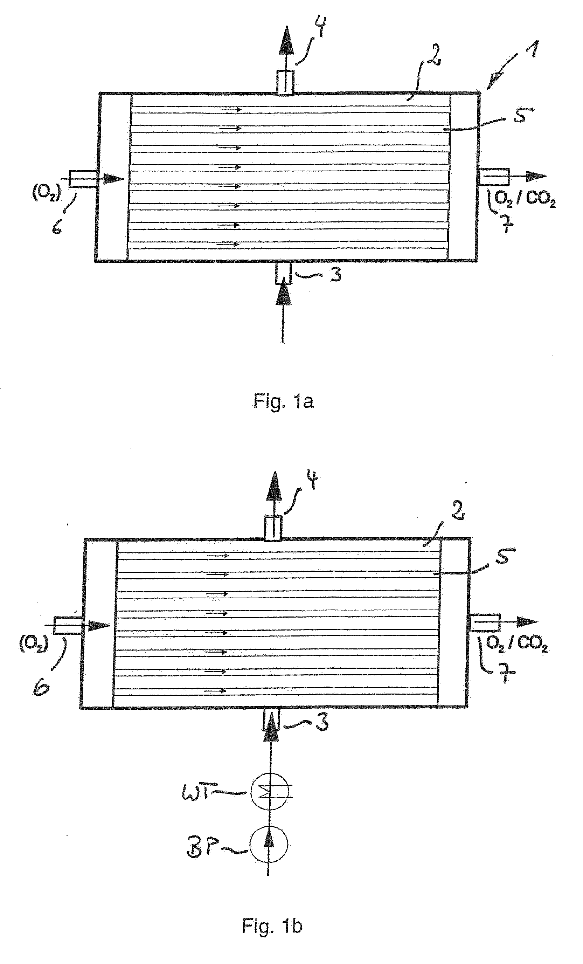

Die Figur 1 zeigt den bekannten schematischen Aufbau eines Blut-Oxygenators, umfassend eine Vorrichtung 1 mit einer Kammer 2, weiche einen Bluteingang 3 und einen Blutausgang 4 aufweist. Diese Kammer kann demnach durch Bluteingang und Blutausgang von Blut durchströmt werden, hier im Wesentlichen quer zur Längserstreckung der Vorrichtung 1.

In Richtung der Längserstreckung ist die Kammer 2 von mehreren stoff-permeablen, nämlich hier insbesondere sauerstoff- und kohlendioxid-permeablen Hohlfasern 5 in axialer Richtung durchzogen, so dass durch den linksseitigen Gaseingang 6 Sauerstoff in die Vorrichtung hinzugefügt werden kann, der durch den rechtsseitigen Gasausgang 7 die Vorrichtung verlässt. Wird demnach durch eine hier nicht dargestellte externe Pumpe, verbrauchtes, d.h. mit CO₂ angereichertes Blut in die Kammer 2 hineingepumpt, so erfolgt auf Grund der hohen Partialdruckunterschiede beiderseits der Hohlfasern 5 ein Gasaustausch, bei dem CO₂ aus dem Blut in die Gasphase transferiert wird und Sauerstoff aus der Gasphase in das Blut transferiert wird. Es kann so bei kontinuierlicher Pumpfunktion verbrauchtes Blut aus dem Körper eines Patienten mit Sauerstoff angereichert und in den Körper des Patienten zurückgepumpt werden.

Die hier dargestellte Ausführung eines bekannten klassischen Blut-Oxygenators hat dabei den Nachteil einer großen Bauform sowie der Notwendigkeit einer externen Pumpe.

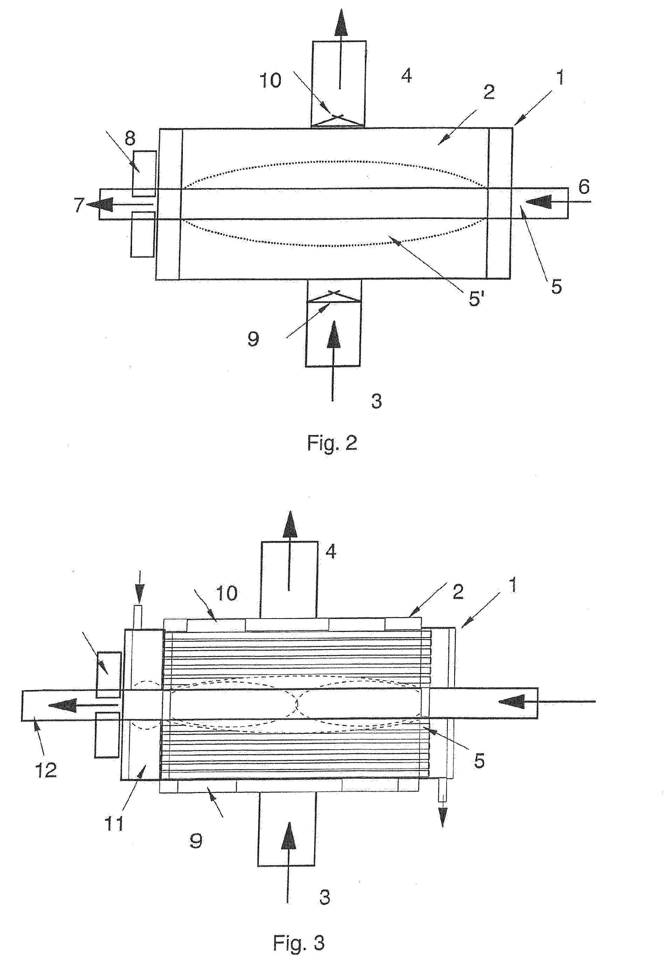

Die Figur 2 zeigt eine einfache Ausführung eines erfindungsgemäß aufgebauten Blut-Oxygenators, der wiederum als eine Vorrichtung 1 ausgebildet ist, die eine innere Kammer 2 aufweist mit einem Bluteingang 3 und einem Blutausgang 4. Quer zur Strömungsrichtung des Blutes verläuft eine elastisch verformbare und gleichzeitig stoff- und/oder energie-permeable Hohlfaser 5 durch die Kammer 2, wobei die Hohlfaser 5, hier in der Anwendung eines Blut-Oxygenators, von Sauerstoff durchströmt wird, nämlich hier vom rechtsseitigen Gaseingang 6 zum linksseitigen Gasausgang 7.

Hierbei wird der Sauerstoff durch den rechtsseitigen Gaseingang kontinuierlich zugeführt und es erfolgt eine Steuerung des in der Hohlfaser 5 wirkenden Druckes durch eine in dem Bereich des Gasausganges angeordnete Drucksteuerung 8, die beispielsweise derart ausgebildet sein kann, dass der wirksame Querschnitt der Hohlfaser 5 verringert bzw. erweitert werden kann.

Wird hier der Querschnitt durch die Drucksteuerung 8 verringert, so steigt der Druck in der Hohlfaser 5 an, so dass diese sich aufbläht und ein größeres Volumen einnimmt, wodurch es im Bereich 5', der durch die Oberfläche der aufgeblähten Hohlfaser 5 definiert wird, zu einer Verdrängung von Blut aus der Kammer 2 kommt. Um hier eine definierte Fließrichtung, nämlich vom Bluteingang 3 zum Blutausgang 4 zu realisieren, ist es vorgesehen, am Bluteingang 3 ein Einlassventil 9 und am Blutausgang 4 ein Auslassventil 10 vorzusehen, wobei beide Ventile als Einwege-Ventile in der derselben Richtung arbeiten.

So wird sichergestellt, dass bei einer Verdrängung, d.h. bei einem Aufblähen der elastisch verformbaren Hohlfaser 5 Blut nur durch den Blutausgang 4 aus der Kammer 2 herausgedrückt wird und bei einer Verringerung des Volumens der Faser 5 durch den dann entstehenden Unterdruck und das geschlossene Auslassventil 10 neues zu behandelndes Blut durch den Bluteingang 3 und das Einlassventil 9 in die Kammer 2 hinein gesogen wird.

Bei dieser Ausführungsform gemäß der Figur 2 wird somit deutlich, dass hier durch die elastisch verformbare Faser 5 sowohl die Pumpenfunktion innerhalb der Vorrichtung 1 realisiert wird als auch auf Grund der Tatsache, dass diese Faser gleichzeitig stoff- und/oder energie-permeabel ist auch der Stoff-, d. h. hier der Gasaustausch, zwischen der Blut- und der Gasphase stattfinden kann.

Die Figur 3 zeigt demgegenüber eine weitere bevorzugte Ausführung eines Blut-Oxygenators als eine gesamte Vorrichtung 1, in der sowohl die eingangs genannte Kammer 2 als auch eine weitere Kammer 11 angeordnet ist. Der Aufbau ist hier im Wesentlichen vergleichbar mit dem der Figur 2, wobei sich hier jedoch eine zentrale elastisch verformbare Hohlfaser 12, die zur Realisierung der Pumpfunktion, nicht jedoch zum Stoffaustausch dient, sich durch die Kammer 2 und die Kammer 11 hindurch erstreckt.

Hierbei dient die Kammer 11 zur Zuführung des zweiten Mediums im Sinne der Erfindung, das heißt hier bei der Anwendung eines Blut-Oxygenators zur Zuführung von Sauerstoff, der sodann auf eine Vielzahl von stoff-permeablen Hohlfasern 5 verteilt wird, die sich im Wesentlichen quer zum Blutstrom durch die Kammer 2 erstrecken.

So wird auch hier Blut durch einen Bluteingang 3 in die Kammer 2 und aus der Kammer 2 durch den Blutausgang 4 herausgepumpt, wobei hier wiederum Ventile 9,10 zur Definition der Flussrichtung vorgesehen sind. Der wesentliche Unterschied gegenüber der Figur 2 ist hier neben der hohen Anzahl von stoffpermeablen Hohlfasern 5 die um die zentrale, elastisch verformbare Hohlfaser 12 angeordnet sind, das weitere erfindungsgemäße Merkmal, dass die Hohlfaser 12 auch in der Kammer 11 einen elastisch verformbaren Bereich aufweist, so dass bei einer Druckerhöhung innerhalb der Hohlfaser 12 nicht nur in der Kammer 2 Blut verdrängt wird, sondern sich auch eine Druckerhöhung in der Kammer 11 ergibt, die sich bis in die einzelnen stoff-permeablen Hohlfasern 5 fortsetzt.

So ergibt sich neben dem Pumpeffekt in der Kammer 2 durch periodische Druckschwankungen in der Hohlfaser 12 ebenso eine periodische Druckschwankung in der Kammer 11 und im Innern der stoff-permeablen Hohlfasern 5, so dass hier ergänzend die Gasgrenzschicht im inneren der Hohlfasern 5 gestört wird.

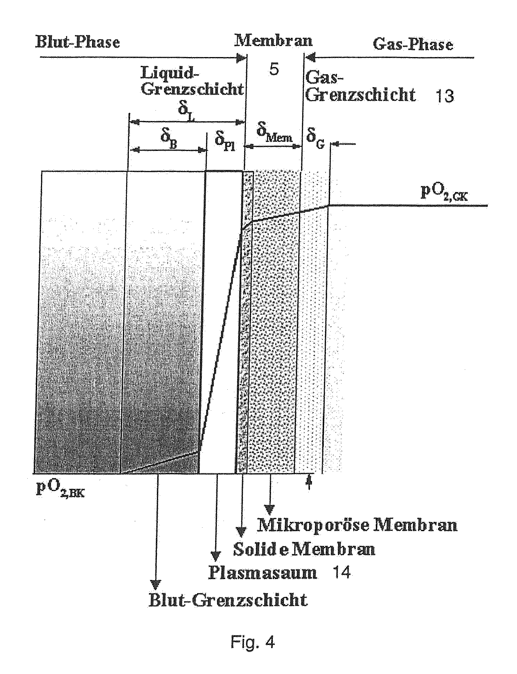

Die wirkenden Verhältnisse bei einem Stoffaustausch in einem Blut-Oxygenator der vorbeschriebenen Art werden beispielsweise in der Figur 4 näher dargestellt. Erkennbar ist hier in der Figur 4 eine Membran 5, die beispielsweise durch die Wandung einer stoff-permeablen Hohlfaser 5 des zuvor genannten Ausführungsbeispiels gegeben sein kann. Von der Innenseite der Hohlfaser wirkt die Gasphase durch einen erhöhten Sauerstoff-Partialdruck pO₂. Unmittelbar angrenzend an die Membran 5 ergibt sich dabei eine Gasgrenzschicht 13, in der der Gasaustausch stattfindet, so dass an dieser Grenzschicht das aus dem Blut aufgenommene CO₂ angereichert wird. Durch diese Anreicherung wird die Effektivität des Gasaustausches, d.h. der Übertrag von Sauerstoff in das Blut, verringert.

Die Ausführungsform gemäß der Figur 3 mit der erzeugten Pulsation in der Kammer 11 bewirkt hier eine Zerstörung dieser Gasgrenzschicht 13, so dass hierdurch leichter Sauerstoff in die Grenzbereiche der Membran 5 kommen kann und hierdurch die Effektivität des gesamten Gasaustausches erhöht wird.

In gleicher Weise bewirkt die intern in der Kammer 2 erzeugte Pumpfunktion periodische Druckschwankungen innerhalb der Kammer 2, die einen Plasmasaum 14, der sich bei Blut-Oxygenatoren blutseitig an der Membran 5 ergibt, gestört wird. Durch die Störung dieses Plasmasaums 14 kann ebenso die Effektivität des Stoffaustausches erhöht werden. Hier zeigt sich in dem eingezeichneten Verlauf des Sauerstoffpartialdruckes, dass die hauptsächlich relevante Stoffübertragung innerhalb des Plasmasaums stattfindet, d.h. hier der Sauerstoffpartialdruck signifikant abfällt von der rechtzeitigen Gasphase zur linksseitigen Blutphase. Gerade die Störung des Plasmasaums durch die in der Kammer 2 erfolgenden Druckschwankungen, die durch die Verformung der elastischen Membran erzeugt werden, kann demnach hier signifikant zur Effizienzsteigerung der Übertragungsraten beitragen.

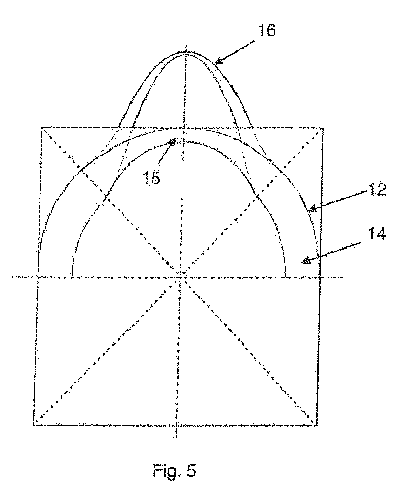

Die Figur 5 zeigt weiterhin eine mögliche Ausführungsform einer elastisch verformbaren Hohlfaser 12. Die hier dargestellte elastisch verformbare Faser 12 hat einen dicken Wandbereich 14 sowie partiell auch einen in der Dicke reduzierten Wandbereich 15, so dass bei einer Druckerhöhung im Inneren dieser Hohlfaser 12 eine Ausbeulung 16 im Wesentlichen dort entstehen wird, wo die Dickenreduktion 15 angeordnet ist. Dort ist die Hohlfaser 12 am stärksten geschwächt, so dass dieser Wandbereich 15 am leichtesten der Druckerhöhung nachgibt. Es kann so mit dieser Ausführungsform erzielt werden, dass durch Anordnung definierter Wandreduktionen sich eine Verformung einer elastischen Hohlfaser 12 nur in einem gewünschten Bereich ergibt, wodurch beispielsweise auch die maximal erreichbare Volumenänderung der Hohlfaser 12 definiert werden kann.

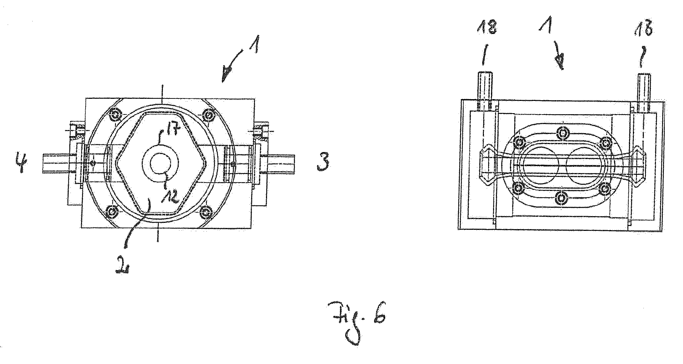

Die Figur 6 zeigt hier ein Beispiel des Gehäuses einer erfindungsgemäßen Vorrichtung. Erkennbar ist linkseitig in Querschnitt die blutdurchflossene Kammer 2 mit einem im Wesentlichen 6-eckigen Querschnitt. Hier sind die nicht dargestellten starren stoffpermeablen Hohlfasern senkrecht zur Blattebene angeordnet und erstrecken sich somit quer zur Strömungsrichtung des Blutes, welches vom Bluteingang 3 zum Blutausgang 4 strömt.

Zentral im Inneren der Kammer ist ein hier zylindrischer Bereich 17 von permeablen Hohlfasern ausgespart und darin zentral eine verformbare elastische Hohlfaser 12 angeordnet um die Pumpfunktion zu realisieren. In der rechtsseitigen Darstellung sind die Anschlüsse 18 dargestellt, um die Hohlfaser mit einem Medium zu beaufschlagen. Die Sauerstoffanschlüsse hin hier nicht dargestellt.

Insgesamt zeigt sich bei der Betrachtung der Erfindung, dass kleinbauende Oxygenatoren realisiert werden können, die sowohl zum Einsatz bei Babys, insbesondere Frühgeborenen vorgesehen werden können sowie auch als implantierbare Organe.

Bezüglich sämtlicher Ausführungsformen ist hier festzustellen, dass die in Verbindung mit einer Ausführung genannten technischen Merkmale nicht nur bei der spezifischen Ausführung eingesetzt werden können, sondern auch bei den jeweils anderen Ausführungen, die im Rahmen dieser Beschreibung genannt oder auch nicht genannt sind. Sämtliche offenbarten technischen Merkmale dieser Erfindungsbeschreibung sind als erfindungswesentlich einzustufen und beliebig miteinander kombinierbar oder in Alleinstellung einsetzbar.

## Patentansprüche

1. Vorrichtung für den Stoff- und/oder Energieaustausch zwischen zwei Medien, insbesondere zwischen Blut und einem Gas/Gasgemisch, mit einer von dem ersten Medium durchströmten Kammer (2), in der wenigstens eine stoff- und/oder energie-permeable Hohlfaser (5), bevorzugt eine Vielzahl von stoff- und/oder energie-permeablen Hohlfasern (5) angeordnet ist, die von dem zweiten Medium durchströmbar und von dem ersten Medium umströmbar ist, **dadurch gekennzeichnet, dass** in der Kammer (2) wenigstens ein Pumpelement (5) angeordnet ist, mittels dem das erste Medium aus der Kammer (2) verdrängbar und/oder in die Kammer (2) hinein saugbar ist.

2. Vorrichtung nach Anspruch 1, **dadurch gekennzeichnet, dass** das Pumpelement (5) als verformbares, insbesondere elastisch verformbares Element angeordnet ist, dessen Oberfläche/Volumen zumindest bereichsweise änderbar ist, so dass bei einer Oberflächen-Nolumenvergrößerung das erste Medium aus der Kammer (2) verdrängbar und bei einer Oberflächen-/Volumenverkleinerung das erste Medium in die Kammer (2) hinein saugbar ist.

3. Vorrichtung nach einem der vorherigen Ansprüche, **dadurch gekennzeichnet, dass** zur Definition einer Flussrichtung in der Kammer (2) am Eingang und am Ausgang für das erste Medium jeweils ein Ventil (9,10), insbesondere ein Einweg-Ventil angeordnet ist.

4. Vorrichtung nach einem der vorherigen Ansprüche, **dadurch gekennzeichnet, dass** das wenigstens eine verformbare Pumpelement (5) als verformbare, insbesondere elastisch verformbare Hohlfaser (5)
ausgebildet ist, deren Oberfläche/Volumen zumindest bereichsweise durch innere Beaufschlagung mit einem Medium änderbar ist.

5. Vorrichtung nach einem der vorherigen Ansprüche, **dadurch gekennzeichnet, dass** die wenigstens eine insbesondere elastisch verformbare Hohlfaser (5) als stoff- und/oder energie-permeable Hohlfaser ausgebildet ist.

6. Vorrichtung nach einem der vorherigen Ansprüche, **dadurch gekennzeichnet, dass** wenigstens ein Teil von allen stoff- und/oder energie-permeablen Hohlfasern (5) als insbesondere elastisch verformbare Hohlfasern (5) ausgebildet ist.

7. Vorrichtung nach einem der vorherigen Ansprüche, **dadurch gekennzeichnet, dass** eine insbesondere elastisch verformbare und stoff- und/oder energie-permeable Hohlfaser (5) als Silikonschlauch ausgebildet ist.

8. Vorrichtung nach einem der vorherigen Ansprüche, **dadurch gekennzeichnet, dass** das Medium zur Beaufschlagung der wenigstens einen elastisch verformbaren Hohlfaser (5) dem zweiten Medium entspricht.

9. Vorrichtung nach einem der vorherigen Ansprüche 1 bis 7, **dadurch gekennzeichnet, dass** das Medium zur Beaufschlagung der wenigstens einen elastisch verformbaren Hohlfaser (5) durch ein Fluid gebildet wird, mittels welchem ein Energieaustausch, insbesondere ein Wärmeenergieaustausch zum erstem Medium stattfindet.

10. Vorrichtung nach einem der vorherigen Ansprüche, **dadurch gekennzeichnet, dass** zusätzlich zu der genannten Kammer (2), die vom ersten Medium durchströmt ist, wenigstens eine weitere Kammer (11) vorgesehen ist, über welche das zweiten Medium der wenigstens einen stoff- und/oder energie-permeablen Hohlfaser (5), bevorzugt der Vielzahl
von stoff- und/oder energie-permeablen Hohlfasern (5) zugeführt ist, wobei sich die wenigstens eine insbesondere elastisch verformbare Hohlfaser (5) durch die genannte (2) und die wenigstens eine weitere Kammer (11) erstreckt.

11. Vorrichtung nach Anspruch 10, **dadurch gekennzeichnet, dass** die Oberfläche/das Volumen der wenigstens einen insbesondere elastisch verformbaren Hohlfaser (5) auch in der wenigstens einen weiteren Kammer (11) durch innere Beaufschlagung mit dem Medium zumindest bereichsweise änderbar ist, so dass bei einer Oberflächen-/Volumenänderung Druckschwankungen in dem zweiten Medium erzeugbar sind.

12. Vorrichtung nach einem der vorherigen Ansprüche, **dadurch gekennzeichnet, dass** Oberflächen-/Volumenänderungen der wenigstens einen insbesondere elastisch verformbaren Hohlfaser (5) durch Druckänderungen des durch diese Hohlfaser fließenden Mediums erzeugbar sind.

13. Vorrichtung nach Anspruch 12, **dadurch gekennzeichnet, dass** die Druckänderungen erzeugbar sind durch einen kontinuierlichen Zufluss des Mediums in die wenigstens eine insbesondere elastisch verformbare Hohlfaser am Eintritt in die Vorrichtung und einen in der Menge steuerbaren Abfluss am Austritt der Vorrichtung, insbesondere durch einen gesteuert änderbaren Querschnitt.

14. Vorrichtung nach einem der vorherigen Ansprüche, **dadurch gekennzeichnet, dass** zur Erzeugung wenigstens eines lokal begrenzten Bereiches der Oberflächen-/Volumenänderung eine verformbare Hohlfaser (5) wenigstens ein sie umgebendes Stützelement aufweist, insbesondere wenigstens einen Ring oder ein Rohr oder ein Gitter.

15. Vorrichtung nach einem der vorherigen Ansprüche, **dadurch gekennzeichnet, dass** zur Erzeugung wenigstens eines lokal begrenzten
Bereiches der Oberflächen-/Volumenänderung eine elastische Hohlfaser (5) eine bereichsweise variable Wandstärke und/oder eine Oberflächenprofilierung aufweist.

16. Vorrichtung nach einem der vorherigen Ansprüche, **dadurch gekennzeichnet, dass** wenigstens eine elastisch vorformbare Hohlfaser (5) von einer Vielzahl von nicht elastischen und stoff- und/oder energiepermeablen Hohlfasern insbesondere symmetrisch umgeben ist.

17. Vorrichtung nach Anspruch 16, **dadurch gekennzeichnet, dass** die Gesamtanordnung von Hohlfasern (5) einen im wesentlichen mehreckigen, insbesondere sechseckigen Querschnitt aufweist.

18. Vorrichtung nach einem der vorherigen Ansprüche 16 oder 17, **dadurch gekennzeichnet, dass** die nicht verformbaren und stoff- und/oder energiepermeablen Hohlfasern (5) versetzt zueinander, insbesondere um einen halben Abstand zwischen zwei Hohlfasern versetzt geschichtet sind.

19. Vorrichtung nach einem der vorherigen Ansprüche, **dadurch gekennzeichnet, dass** sie ein Füllvolumen des ersten Mediums aufweist, welches geringer ist als das Blutvolumen eines Säuglings, insbesondere weniger als 100 ml.

20. Verwendung einer Vorrichtung nach einem der vorherigen Ansprüche als Oxygenator, insbesondere Blut-Oxygenator, insbesondere für Früh- und Neugeborene sowie Kleinkinder und Erwachsene, insbesondere als implantierbarer Oxygenator.

21. Verwendung einer Vorrichtung nach einem der vorherigen Ansprüche als implantierbares künstliches Organ, insbesondere als Dialysegerät.
